(19) Europäisches Patentamt
European Patent Office
Office européen des brevets

(11) **EP 1 889 569 B1**

(12) **EUROPEAN PATENT SPECIFICATION**

(45) Date of publication and mention
of the grant of the patent:
**18.06.2014  Bulletin 2014/25**

(51) Int Cl.:
***A61B 5/00*** (2006.01)

(21) Application number: **07021807.8**

(22) Date of filing: **25.01.2000**

(54) **Universal/upgrading pulse oximeter**

Universelles/Aktualisierendes Pulsoximeter

Sphygmo-oxymètre universel à fonction d'amélioration

(84) Designated Contracting States:
**AT BE CH CY DE DK ES FI FR GB GR IE IT LI LU
MC NL PT SE**

(30) Priority: **25.01.1999   US 117097 P**
**26.10.1999   US 161565 P**

(43) Date of publication of application:
**20.02.2008   Bulletin 2008/08**

(60) Divisional application:
**08012674.1 / 1 992 278**

(62) Document number(s) of the earlier application(s) in
accordance with Art. 76 EPC:
**00907031.9 / 1 148 809**

(73) Proprietor: **Masimo Corporation
Irvine, CA 92618 (US)**

(72) Inventors:
• **Kiani, Massi, E.**
**Laguna Niguel, CA 92677 (US)**
• **Ali, Ammar, Al**
**Tustin, CA 92782 (US)**
• **Diab, Mohamed, K.**
**Mission Viejo, CA 92692 (US)**
• **Vaden, Val, E.**
**Hillsborough, CA 94010 (US)**

(74) Representative: **Vossius & Partner
Siebertstrasse 4
81675 München (DE)**

(56) References cited:
WO-A-96/16591      US-A- 5 343 869
US-A- 5 375 604      US-A- 5 687 717
US-A- 5 931 791

## Description

Oximetry is the measurement of the oxygen level status of blood. Early detection of low blood oxygen level is critical in the medical field, for example in critical care and surgical applications, because an insufficient supply of oxygen can result in brain damage and death in a matter of minutes. Pulse oximetry is a widely accepted noninvasive procedure for measuring the oxygen saturation level of arterial blood, an indicator of oxygen supply. A pulse oximetry system consists of a sensor applied to a patient, a pulse oximeter, and a patient cable connecting the sensor and the pulse oximeter.

The pulse oximeter may be a standalone device or may be incorporated as a module or built-in portion of a multiparameter patient monitoring system, which also provides measurements such as blood pressure, respiratory rate and EKG. A pulse oximeter typically provides a numerical readout of the patient's oxygen saturation, a numerical readout of pulse rate, and an audible indicator or "beep" that occurs in response to each pulse. In addition, the pulse oximeter may display the patient's plethysmograph, which provides a visual display of the patient's pulse contour and pulse rate.

US 5,687,717 discloses a patient monitoring system including one or more chassis, a plurality of patient care modules associated with the chassis, and a portable computer for communicating with and controlling the modules. Each patient is assigned to a chassis, and each module is assigned to a patient and to a chassis. Each module is fully operational in either an independent or a dependent mode. In both modes, the chassis continuously polls the module for patient data collected by the module. In the dependent mode, the module is physically received by, and powered from, the chassis, and sends patient data to a computer in the chassis for storage therein. In the independent mode, the module is remote from, and independent of, the chassis. In the independent mode, the module stores patient data in its own memory when data cannot be communicated to the chassis. When requested by the portable computer, the chassis communicate the patient data to the portable computer. The portable computer may be connected to the chassis or may also be used remote from the chassis.

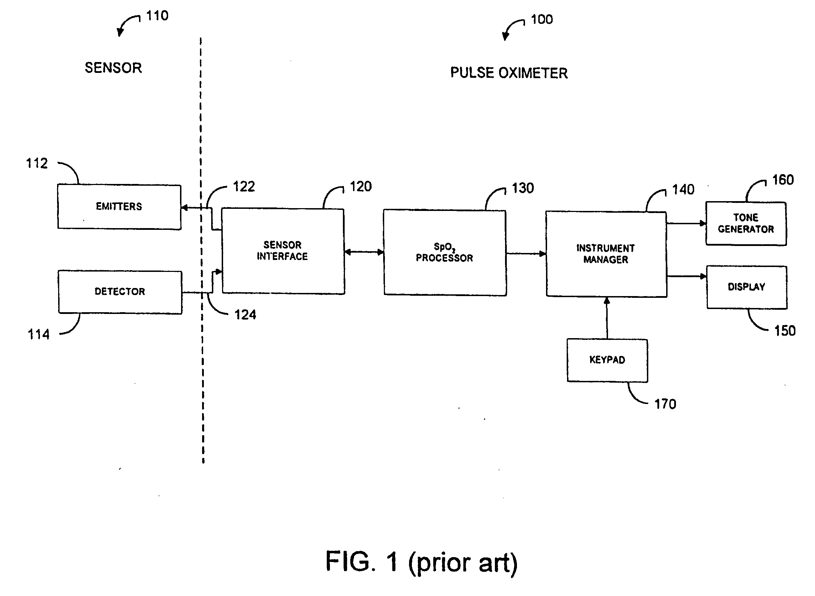

FIG. **1** illustrates a prior art pulse oximeter **100** and associated sensor **110**. Conventionally, a pulse oximetry sensor **110** has LED emitters **112**, typically one at a red wavelength and one at an infrared wavelength, and a photodiode detector **114**. The sensor **110** is typically attached to an adult patient's finger or an infant patient's foot. For a finger, the sensor **110** is configured so that the emitters **112** project light through the fingernail and through the blood vessels and capillaries underneath. The LED emitters **112** are activated by drive signals **122** from the pulse oximeter **100**. The detector **114** is positioned at the fingertip opposite the fingernail so as to detect the LED emitted light as it emerges from the finger tissues. The photodiode generated signal **124** is relayed by a cable to the pulse oximeter **100**.

The pulse oximeter **100** determines oxygen saturation ($SpO_2$) by computing the differential absorption by arterial blood of the two wavelengths emitted by the sensor **110**. The pulse oximeter **100** contains a sensor interface **120**, an $SpO_2$ processor **130**, an instrument manager **140,** a display **150,** an audible indicator (tone generator) **160** and a keypad **170**. The sensor interface **120** provides LED drive current **122** which alternately activates the sensor red and IR LED emitters **112.** The sensor interface **120** also has input circuitry for amplification and filtering of the signal **124** generated by the photodiode detector **114,** which corresponds to the red and infrared light energy attenuated from transmission through the patient tissue site. The $SpO_2$ processor **130** calculates a ratio of detected red and infrared intensities, and an arterial oxygen saturation value is empirically determined based on that ratio. The instrument manager **140** provides hardware and software interfaces for managing the display **150**, audible indicator **160** and keypad **170**. The display **150** shows the computed oxygen status, as described above. The audible indicator **160** provides the pulse beep as well as alarms indicating desaturation events. The keypad **170** provides a user interface for such things as alarm thresholds, alarm enablement, and display options.

Computation of $SpO_2$ relies on the differential light absorption of oxygenated hemoglobin, $HbO_2$, and deoxygenated hemoglobin, $Hb$, to determine their respective concentrations in the arterial blood. Specifically, pulse oximetry measurements are made at red and IR wavelengths chosen such that deoxygenated hemoglobin absorbs more red light than oxygenated hemoglobin, and, conversely, oxygenated hemoglobin absorbs more infrared light than deoxygenated hemoglobin, for example 660 nm (red) and 905 nm (IR).

To distinguish between tissue absorption at the two wavelengths, the red and IR emitters **112** are provided drive current **122** so that only one is emitting light at a given time. For example, the emitters **112** may be cycled on and off alternately, in sequence, with each only active for a quarter cycle and with a quarter cycle separating the active times. This allows for separation of red and infrared signals and removal of ambient light levels by downstream signal processing. Because only a single detector **114** is used, it responds to both the red and infrared emitted light and generates a time-division-multiplexed ("modulated") output signal **124**. This modulated signal **124** is coupled to the input of the sensor interface **120**.

In addition to the differential absorption of hemoglobin derivatives, pulse oximetry relies on the pulsatile nature of arterial blood to differentiate hemoglobin absorption from absorption of other constituents in the surrounding tissues. Light absorption between systole and diastole varies due to the blood volume change from the inflow and outflow of arterial blood at a peripheral tissue site. This tissue site might also comprise skin, muscle, bone, venous blood, fat, pigment, etc., each of which absorbs light. It is assumed that the background absorption due to these surrounding tissues is invariant and can be ignored. Thus, blood oxygen saturation measurements are based upon a ratio of the time-varying or AC portion of the detected red and infrared signals with respect to the time-invariant or DC portion:

$$RD/IR = (Red_{AC}/Red_{DC})/(IR_{AC}/IR_{DC})$$

The desired $SpO_2$ measurement is then computed from this ratio. The relationship between RD/IR and $SpO_2$ is most accurately determined by statistical regression of experimental measurements obtained from human volunteers and calibrated measurements of oxygen saturation. In a pulse oximeter device, this empirical relationship can be stored as a "calibration curve" in a read-only memory (ROM) look-up table so that $SpO_2$ can be directly read-out of the memory in response to input RD/IR measurements.

Pulse oximetry is the standard-of-care in various hospital and emergency treatment environments. Demand has lead to pulse oximeters and sensors produced by a variety of manufacturers. Unfortunately, there is no standard for either performance by, or compatibility between, pulse oximeters or sensors. As a result, sensors made by one manufacturer are unlikely to work with pulse oximeters made by another manufacturer. Further, while conventional pulse oximeters and sensors are incapable of taking measurements on patients with poor peripheral circulation and are partially or fully disabled by motion artifact, advanced pulse oximeters and sensors manufactured by the assignee of the present invention are functional under these conditions. This presents a dilemma to hospitals and other caregivers wishing to upgrade their patient oxygenation monitoring capabilities. They are faced with either replacing all of their conventional pulse oximeters, including multiparameter patient monitoring systems, or working with potentially incompatible sensors and inferior pulse oximeters manufactured by various vendors for the pulse oximetry equipment in use at the installation.

Hospitals and other caregivers are also plagued by the difficulty of monitoring patients as they are transported from one setting to another. For example, a patient transported by ambulance to a hospital emergency room will likely be unmonitored during the transition from ambulance to the ER and require the removal and replacement of incompatible sensors in the ER. A similar problem is faced within a hospital as a patient is moved between surgery, ICU and recovery settings. Incompatibility and transport problems are exacerbated by the prevalence of expensive and non-portable multiparameter patient monitoring systems having pulse oximetry modules as one measurement parameter.

The Universal/Upgrading Pulse Oximeter (UPO) according to the present invention is focused on solving these performance, incompatibility and transport problems. The UPO provides a transportable pulse oximeter that can stay with and continuously monitor the patient as they are transported from setting to setting. Further, the UPO provides a synthesized output that drives the sensor input of other pulse oximeters. This allows the UPO to function as a universal interface that matches incompatible sensors with other pulse oximeter instruments. Further, the UPO acts as an upgrade to existing pulse oximeters that are adversely affected by low tissue perfusion and motion artifact. Likewise, the UPO can drive a $SpO_2$ sensor input of multiparameter patient monitoring systems, allowing the UPO to integrate into the associated multiparameter displays, patient record keeping systems and alarm management functions.

The present invention relates to a physiological measurement system according to claim 1.

One aspect of the present invention is a measurement apparatus comprising a sensor, a first pulse oximeter and a waveform generator. The sensor has at least one emitter and an associated detector configured to attach to a tissue site. The detector provides an intensity signal responsive to the oxygen content of arterial blood at the tissue site. The first pulse oximeter is in communication with the detector and computes an oxygen saturation measurement based on the intensity signal. The waveform generator is in communication with the first pulse oximeter and provides a waveform based on the oxygen saturation measurement. A second pulse oximeter is in communication with the waveform generator and displays an oxygen saturation value based on the waveform. The waveform is synthesized so that the oxygen saturation value is generally equivalent to the oxygen saturation measurement.

In another example, a measurement apparatus comprises a first sensor port connectable to a sensor, an upgrade port, a signal processor and a waveform generator. The upgrade port is connectable to a second sensor port of a physiological monitoring apparatus. The signal processor is configured to compute a physiological measurement based on a signal input to the first sensor port. The waveform generator produces a waveform based on the physiological measurement, and the waveform is available at the upgrade port. The waveform is adjustable so that the physiological monitoring apparatus displays a value generally equivalent to the physiological measurement when the upgrade port is attached to the second sensor port.

Yet another example is a measurement method comprising the steps of sensing an intensity signal responsive to the oxygen content of arterial blood at a tissue site and computing an oxygen saturation measurement based on the intensity signal. Other steps are generating a waveform based on the oxygen saturation measurement and providing the waveform to the sensor inputs of a pulse oximeter so that the pulse oximeter displays an oxygen saturation value generally equivalent to the oxygen saturation measurement.

An additional example is a measurement method comprising the steps of sensing a physiological signal, computing a physiological measurement based upon the signal, and synthesizing a waveform as a function of the physiological measurement. A further step is outputting the waveform to a sensor input of a physiological monitoring apparatus. The synthesizing step is performed so that the measurement apparatus displays a value corresponding to the physiological measurement.

A further example is a measurement apparatus comprising a first pulse oximeter for making an oxygen saturation measurement and a pulse rate measurement based upon an intensity signal derived from a tissue site. Also included is a waveform generation means for creating a signal based upon the oxygen saturation measurement and the pulse rate measurement. In addition, there is a communication means for transmitting the signal to a second pulse oximeter.

Another example is a measurement apparatus comprising a portable portion having a sensor port, a processor, a display, and a docking connector. The sensor port is configured to receive an intensity signal responsive to the oxygen content of arterial blood at a tissue site. The processor is programmed to compute an oxygen saturation value based upon the intensity signal and to output the value to the display. A docking station has a portable connector and is configured to accommodate the portable so that the docking connector mates with the portable connector. This provides electrical connectivity between the docking station and the portable. The portable has an undocked position separate from the docking station in which the portable functions as a handheld pulse oximeter. The portable also has a docked position at least partially retained within the docking station n in which the combination of the portable and the docking station has at least one additional function compared with the portable in the undocked position.

A further example is a measurement apparatus configured to function in both a first spatial orientation and a second spatial orientation. The apparatus comprises a sensor port configured to receive a signal responsive to a physiological state. The apparatus also has a tilt sensor providing an output responsive to gravity. In addition, there is a processor in communication with the sensor port and the tilt sensor output. The processor is programmed to compute a physiological measurement value based upon the signal and to determine whether the measurement apparatus is in the first orientation or the second orientation based upon the tilt sensor output. A display has a first mode and a second mode and is driven by the processor. The display shows the measurement value in the first mode when the apparatus is in the first orientation and shows the measurement value in the second mode when the apparatus is in the second orientation.

Another example is a measurement method comprising the steps of sensing a signal responsive to a physiological state and computing physiological measurement based on the signal. Additional steps are determining the spatial orientation of a tilt sensor and displaying the physiological measurement in a mode that is based upon the determining step.

FIG. **1** is a block diagram of a prior art pulse oximeter;
FIG. **2** is a diagram illustrating a patient monitoring system incorporating a universal/upgrading pulse oximeter (UPO) according to the present invention;
FIG. **3** is top level block diagram of a UPO embodiment;
FIG. **4** is a detailed block diagram of the waveform generator portion of the UPO embodiment shown in FIG. **3**;
FIG. **5** is an illustration of a handheld embodiment of the UPO;
FIG. **6** is a top level block diagram of another UPO embodiment incorporating a portable pulse oximeter and a docking station;
FIG. **7** is a detailed block diagram of the portable pulse oximeter portion of FIG. **6**;
FIG. **8A** is an illustration of the portable pulse oximeter user interface, including a keyboard and display;
FIGS. **8B-C** are illustrations of the portable pulse oximeter display showing portrait and landscape modes, respectively;
FIG. **9** is a detailed block diagram of the docking station portion of FIG. **6**;
FIG. **10** is a schematic of the interface cable portion of FIG. **6**;
FIG. **11A** is a front view of an embodiment of a portable pulse oximeter;
FIG. **12** is a block diagram of one embodiment of a local area network interface for a docking station.

Detailed Description of the Preferred Embodiments

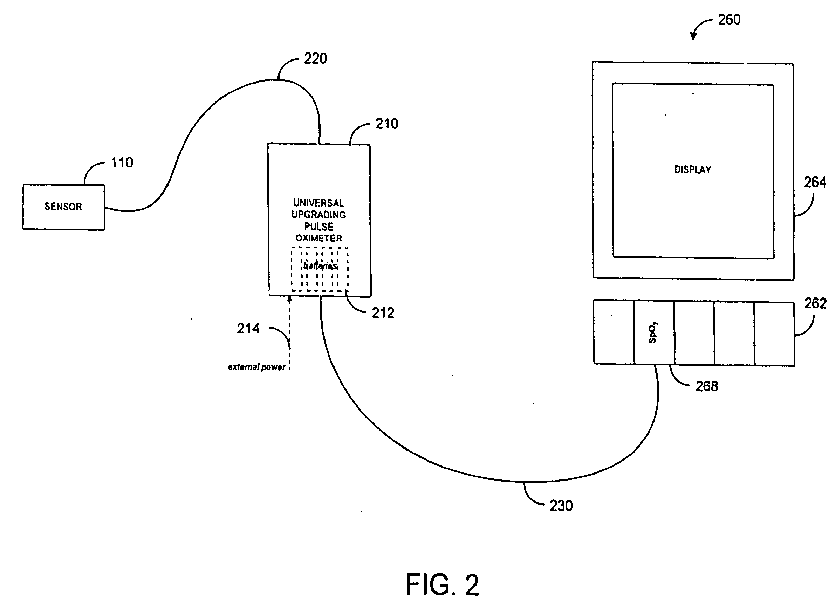

FIG. **2** depicts the use of a Universal/Upgrading Pulse Oximeter ("UPO") **210** to perform patient monitoring. A pulse oximetry sensor **110** is attached to a patient (not illustrated) and provides the UPO **210** with a modulated red and IR photo-plethysmograph signal through a patient cable **220**. The UPO **210** computes the patient's oxygen saturation and pulse rate from the sensor signal and, optionally, displays the patient's oxygen status. The UPO **210** may incorporate an internal power source **212**, such as common alkaline batteries or a rechargeable power source. The UPO **210** may also utilize an external power source **214**, such as standard 110V AC coupled with an external step-down transformer and an internal or external AC-to-DC converter.

In addition to providing pulse oximetry measurements, the UPO **210** also separately generates a signal, which is received by a pulse oximeter **268** external to the UPO **210**. This signal is synthesized from the saturation calculated by the UPO **210** such that the external pulse oximeter **268** calculates the equivalent saturation and pulse rate as computed by the UPO **210**. The external pulse oximeter **268** receiving the UPO signal may be a multiparameter patient monitoring system (MPMS) **260** incorporating a pulse oximeter module **268**, a standalone pulse oximeter instrument, or any other host instrument capable of measuring $SpO_2$. The MPMS **260** depicted in FIG. **2** has a rack **262** containing a number of modules for monitoring such patient parameters as blood pressure, EKG, respiratory gas, and $SpO_2$. The measurements made by these various modules are shown on a multiparameter display **264**, which is typically a video (CRT) device. The UPO **210** is connected to an existing MPMS **260** with a cable **230**, advantageously integrating the UPO oxygen status measurements with other MPMS measurements. This allows the UPO calculations to be shown on a unified display of important patient parameters, networked with other patient data, archived within electronic patient records and incorporated into alarm management, which are all MPMS functions convenient to the caregiver.

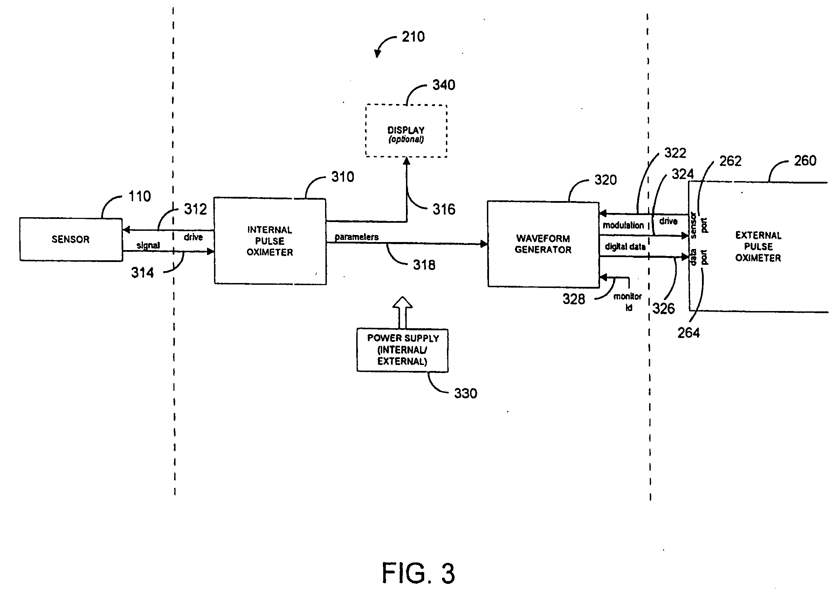

FIG. **3** depicts the major functions of the UPO **210**, including an internal pulse oximeter **310**, a waveform generator **320**, a power supply **330** and an optional display **340**. Attached to the UPO **210** is a sensor **110** and an external pulse oximeter **260**. The internal pulse oximeter **310** provides the sensor **110** with a drive signal **312** that alternately activates the sensor's red and IR LEDs, as is well-known in the art. A corresponding detector signal **314** is received by the internal pulse oximeter **310**. The internal pulse oximeter **310** computes oxygen saturation, pulse rate, and, in some embodiments, other physiological parameters such as pulse occurrence, plethysmograph features and measurement confidence. These parameters **318** are output to the waveform generator **320**. A portion of these parameters may also be used to generate display drive signals **316** so that patient status may be read from, for example, an LED or LCD display module **340** on the UPO.

The internal pulse oximeter **310** may be a conventional pulse oximeter or, for upgrading an external pulse oximeter **260**, it may be an advanced pulse oximeter capable of low perfusion and motion artifact performance not found in conventional pulse oximeters. An advanced pulse oximeter for use as an internal pulse oximeter **310** is described in U.S. Patent No. 5,632,272 assigned to the assignee of the present invention. An advanced pulse oximetry sensor for use as the sensor **110** attached to the internal pulse oximeter **310** is described in U.S. Patent No. 5,638,818 assigned to the assignee of the present invention and incorporated herein by reference. Further, a line of advanced Masimo SET® pulse oximeter OEM boards and sensors are available from the assignee of the present invention.

The waveform generator **320** synthesizes a waveform, such as a triangular waveform having a sawtooth or symmetric triangle shape, that is output as a modulated signal **324** in response to an input drive signal **322**. The drive input **322** and modulation output **324** of the waveform generator **320** are connected to the sensor port **262** of the external pulse oximeter **260**. The synthesized waveform is generated in a manner such that the external pulse oximeter **260** computes and displays a saturation and a pulse rate value that is equivalent to that measured by the internal pulse oximeter **310** and sensor **110**. In the present embodiment, the waveforms for pulse oximetry are chosen to indicate to the external pulse oximeter **260** a perfusion level of 5%. The external pulse oximeter **260**, therefore, always receives a strong signal. In an alternative embodiment, the perfusion level of the waveforms synthesized for the external pulse oximeter can be set to indicate a perfusion level at or close to the perfusion level of the patient being monitored by the internal pulse oximeter **310**. As an alternative to the generated waveform, a digital data output **326**, is connected to the data port **264** of the external pulse oximeter **260**. In this manner, saturation and pulse rate measurements and also samples of the unmodulated, synthesized waveform can be communicated directly to the external pulse oximeter **260** for display, bypassing the external pulse oximeter's signal processing functions. The measured plethysmograph waveform samples output from the internal pulse oximeter **310** also may be communicated through the digital data output **326** to the external pulse oximeter **260**.

It will be understood from the above discussion that the synthesized waveform is not physiological data from the patient being monitored by the internal pulse oximeter **310**, but is a waveform synthesized from predetermined stored waveform data to cause the external pulse oximeter **260** to calculate oxygen saturation and pulse rate equivalent to or generally equivalent (within clinical significance) to that calculated by the internal pulse oximeter **310**. The actual physiological waveform from the patient received by the detector is not provided to the external pulse oximeter **260** in the present embodiment. Indeed, the waveform provided to the external pulse oximeter will usually not resemble the plethysmographic waveform of physiological data from the patient being monitored by the internal pulse oximeter **260**.

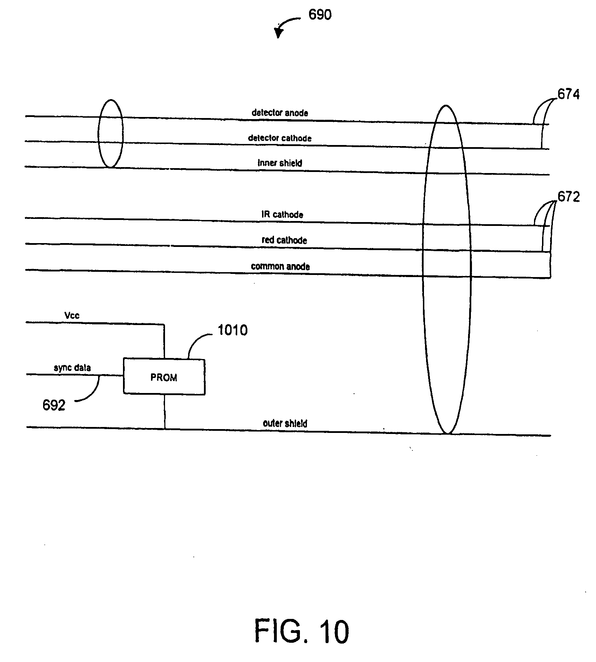

The cable **230** (FIG. **2**) attached between the waveform generator **320** and external pulse oximeter **260** provides a monitor ID **328** to the UPO, allowing identification of predetermined external pulse oximeter calibration curves. For example, this cable may incorporate an encoding device, such as a resistor, or a memory device, such as a PROM **1010** (FIG. **10**) that is read by the waveform generator **320**. The encoding device provides a value that uniquely identifies a particular type of external pulse oximeter **260** having known calibration curve, LED drive and modulation signal characteristics. Although the calibration curves of the external pulse oximeter **260** are taken into account, the wavelengths of the actual sensor **110**, advantageously, are not required to correspond to the particular calibration curve indicated by the monitor ID **328** or otherwise assumed for the external pulse oximeter **260.** That is, the wavelength of the sensor **110** attached to the internal pulse oximeter **310** is not relevant or known to the external pulse oximeter **260.**

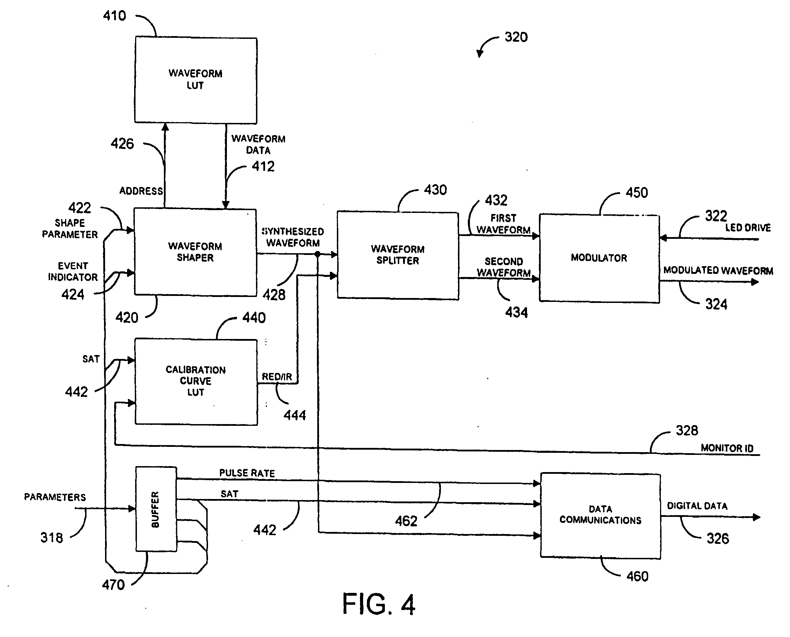

FIG. **4** illustrates one embodiment of the waveform generator portion **320** of the UPO **210** (FIG. **3**). Although this embodiment is illustrated and described as hardware, one of ordinary skill will recognize that the functions of the waveform generator may be implemented in software or firmware or a combination of hardware, software and firmware. The waveform generator **320** performs waveform synthesis with a waveform look-up table ("LUT") **410**, a waveform shaper **420** and a waveform splitter **430**. The waveform LUT **410** is advantageously a memory device, such as a ROM (read only memory) that contains samples of one or more waveform portions or segments containing a single waveform. These stored waveform segments may be as simple as a single period of a triangular waveform, having a sawtooth or symmetric triangle shape, or more complicated, such as a simulated plethysmographic pulse having various physiological features, for example rise time, fall time and dicrotic notch.

The waveform shaper **420** creates a continuous pulsed waveform from the waveform segments provided by the waveform LUT **410**. The waveform shaper **420** has a shape parameter input **422** and an event indicator input **424** that are buffered **470** from the parameters **318** output from the internal pulse oximeter **310** (FIG. **3**). The shape parameter input **422** determines a particular waveform segment in the waveform LUT **410.** The chosen waveform segment is specified by the first address transmitted to the waveform LUT **410** on the address lines **426.** The selected waveform segment is sent to the waveform shaper **420** as a series of samples on the waveform data lines **412.**

The event indicator input **424** specifies the occurrence of pulses in the plethysmograph waveform processed by the internal pulse oximeter **310** (FIG. **3**). For example, the event indicator may be a delta time from the occurrence of a previously detected falling pulse edge or this indicator could be a real time or near real time indicator of the pulse occurrence. The waveform shaper **420** accesses the waveform LUT **410** in a manner to create a corresponding delta time between pulses in the synthesized waveform output **428**. In one embodiment, the waveform shaper is clocked at a predetermined sample rate. From a known number of samples per stored waveform segment and the input delta time from the event indicator, the waveform shaper **420** determines the number of sequential addresses to skip between samples and accesses the waveform LUT **410** accordingly. This effectively "stretches" or "shrinks" the retrieved waveform segment so as to fit in the time between two consecutive pulses detected by the UPO.

The waveform splitter **430** creates a first waveform **432** corresponding to a first waveform (such a red wavelength) expected by the external pulse oximeter **260** (Fig. **3**) and a second waveform (such as infrared) **434** expected by the external pulse oximeter **260**. The relative amplitudes of the first waveform **432** and second waveform **434** are adjusted to correspond to the ratio output **444** from a calibration curve LUT **440**. Thus, for every value of measured oxygen saturation at the sat input **442**, the calibration curve LUT **440** provides a corresponding ratio output **444** that results in the first waveform **432** and the second waveform **434** having an amplitude ratio that will be computed by the external pulse oximeter **260** (FIG. **3**) as equivalent to the oxygen saturation measured by the internal pulse oximeter **310** (FIG. **3**).

As described above, one particularly advantageous aspect of the UPO is that the operating wavelengths of the sensor **110** (FIG. **3**) are not relevant to the operating wavelengths required by the external pulse oximeter **260** (FIG. **3**), i.e. the operating wavelengths that correspond to the calibration curve or curves utilized by the external pulse oximeter. The calibration curve LUT **440** simply permits generation of a synthesized waveform as expected by the external oximeter **260** (FIG. **3**) based on the calibration curve used by the external pulse oximeter **260** (FIG. **3**). The calibration curve LUT **440** contains data about the known calibration curve of the external pulse oximeter **260** (FIG. **3**), as specified by the monitor ID input **328**. In other words, the waveform actually synthesized is not a patient plethysmographic waveform. It is merely a stored waveform that will cause the external pulse oximeter to calculate the proper oxygen saturation valve and pulse rate values.. Although this does not provide a patient plethysmograph on the external pulse oximeter for the clinician, the calculated values, which is what is actually sought, will be accurate.

A modulator **450** responds to an LED drive input **322** to generate a modulated waveform output **324** derived from the first waveform **432** and second waveform **434**. Also, a data communication interface **460** transmits as a digital data output **326** the data obtained from the sat **442**, pulse rate **462** and synthesized waveform **428** inputs.

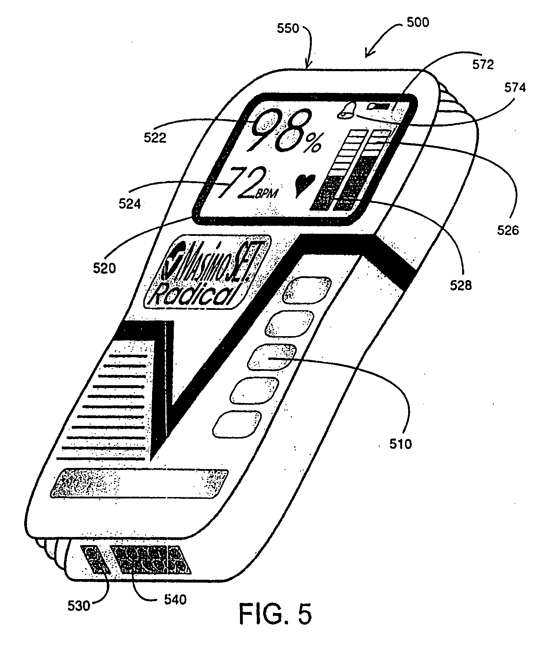

FIG. **5** depicts a handheld UPO **500** embodiment. The handheld UPO **500** has keypad inputs **510**, an LCD display **520**, an external power supply input **530**, an output port **540** for connection to an external pulse oximeter and a sensor input **550** at the top edge (not visible). The display **520** shows the measured oxygen saturation **522**, the measured pulse rate **524**, a pulsating bar **526** synchronized with pulse rate or pulse events, and a confidence bar **528** indicating confidence in the measured values of saturation and pulse rate. Also shown are low battery **572** and alarm enabled **574** status indicators.

The handheld embodiment described in connection with FIG. **5** may also advantageously function in conjunction with a docking station that mechanically accepts, and electrically connects to, the handheld unit. The docking station may be co-located with a patient monitoring system and connected to a corresponding SpO$_2$ module sensor port, external power supply, printer and telemetry device, to name a few options. In this configuration, the handheld UPO may be removed from a first docking station at one location to accompany and continuously monitor a patient during transport to a second location. The handheld UPO can then be conveniently placed into a second docking station upon arrival at the second location, where the UPO measurements are displayed on the patient monitoring system at that location.

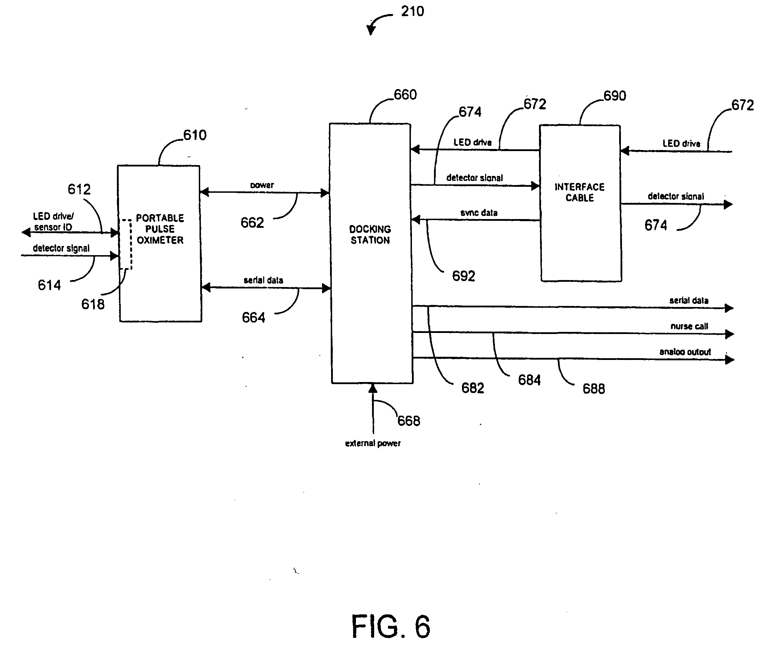

FIG. **6** shows a block diagram of a UPO embodiment, where the functions of the UPO **210** are split between a portable pulse oximeter **610** and a docking station **660.** The portable pulse oximeter **610** ("portable") is a battery operated, fully functional, stand-alone pulse oximeter instrument. The portable **610** connects to a sensor **110** (FIG. **2**) through a UPO patient cable **220** (FIG. **2**) attached to a patient cable connector **618.** The portable **610** provides the sensor **110** with a drive signal **612** that alternately activates the sensor's red and IR LEDs, as is well-known in the art. The portable also receives a corresponding detector signal **614** from the sensor. The portable can also input a sensor ID on the drive signal line **612,** as described in U.S. Patent No. 5,758,644 entitled Manual and Automatic Probe Calibration, assigned to the assignee of the present invention.

The portable **610** can be installed into the docking station **660** to expand its functionality. When installed, the portable **610** can receive power **662** from the docking station **660** if the docking station **660** is connected to external power **668.** Alternately, with no external power **668** to the docking station **660**, the portable **610** can supply power **662** to the docking station **660.** The portable **610** communicates to the docking station with a bidirectional serial data line **664.** In particular, the portable **610** provides the docking station with $SpO_2$, pulse rate and related parameters computed from the sensor detector signal **614.** When the portable **610** is installed, the docking station **660** may drive a host instrument **260** (FIG. **2**) external to the portable **610.** Alternatively, the portable **610** and docking station **660** combination may function as a standalone pulse oximeter instrument, as described below with respect to FIG. **13.**

In one embodiment, the docking station **660** does not perform any action when the portable **610** is not docked. The user interface for the docking station **660**, i.e. keypad and display, is on the portable **610.** An indicator LED on the docking station **660** is lit when the portable is docked. The docking station **660** generates a detector signal output **674** to the host instrument **260** (FIG. **2**) in response to LED drive signals **672** from the host instrument and $SpO_2$ values and related parameters received from the portable **610.** The docking station **660** also provides a serial data output **682,** a nurse call **684** and an analog output **688.**

An interface cable **690** connects the docking station **660** to the host instrument patient cable **230** (FIG. **2**). The LED drive signals **672** and detector signal output **674** are communicated between the docking station **660** and the host instrument **260** (FIG. **2**) via the interface cable **690.** The interface cable **690** provides a sync data output **692** to the docking station **660**, communicating sensor, host instrument (e.g. monitor ID **328**, FIG. **3**) and calibration curve data. Advantageously, this data allows the docking station **660** to appear to a particular host instrument as a particular sensor providing patient measurements.

FIG.1 **7** provides further detail of the portable **610.** The portable components include a pulse oximeter processor **710**, a management processor **720**, a power supply **730**, a display **740** and a keypad **750.** The pulse oximeter processor **710** functions as an internal pulse oximeter, interfacing the portable to a sensor **110** (FIG. **2**) and deriving $SpO_2$, pulse rate, a plethysmograph and a pulse indicator. An advanced pulse oximeter for use as the pulse oximeter processor **710** is described in U.S. Patent No. 5,632,272, referenced above. An advanced pulse oximetry sensor for use as the sensor **110** (FIG. **2**) attached to the pulse oximeter processor **710** is described in U.S. Patent No. 5,638,818, also referenced above. Further, a line of advanced Masimo SET® pulse oximeter OEM boards and sensors are available from the assignee of the present invention. In one embodiment, the pulse oximeter processor **710** is the Masimo SET® MS-3L board or a low power MS-5 board.

The management processor **720** controls the various functions of the portable **610,** including asynchronous serial data communications **724** with the pulse oximeter processor **710** and synchronous serial communications **762** with the docking station **660** (FIG. **6**). The physical and electrical connection to the docking station **660** (FIG. **6**) is via a docking station connector **763** and the docking station interface **760**, respectively. The processor **720** utilizes a real-time clock **702** to keep the current date and time, which includes time and date information that is stored along with $SpO_2$ parameters to create trend data. The processor of the portable **610** and the docking station **660** (FIG. **6**) can be from the same family to share common routines and minimize code development time.

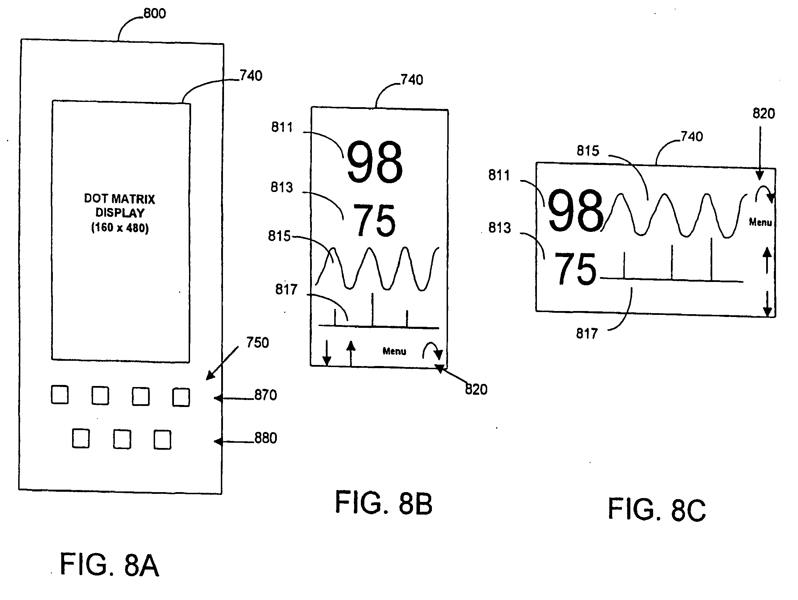

The processor **720** also controls the user interface **800** (FIG. **8A**) by transferring data **742** to the display **740**, including display updates and visual alarms, and by interpreting keystroke data **752** from the keypad **750.** The processor **720** generates various alarm signals, when required, via an enable signal **728**, which controls a speaker driver **770.** The speaker driver **770** actuates a speaker **772**, which provides audible indications such as, for example, alarms and pulse beeps. The processor **720** also monitors system status, which includes battery status **736**, indicating battery levels, and docked status **764,** indicating whether the portable **610** is connected to the docking station **660** (FIG. **6**). When the portable **610** is docked and is on, the processor **720** also decides when to turn on or off docking station power **732.**

Advantageously, the caregiver can set (i.e. configure or program) the behavior of the portable display **740** and alarms when the docked portable **610** senses that an interface cable **690** has connected the docking station **660** to an external pulse oximeter, such as a multiparameter patient monitoring system. In one user setting, for example, the portable display **740** stops showing the SpO$_2$ **811** (FIG. **8**) and pulse rate **813** (FIG. **8**) values when connected to an external pulse oximeter to avoid confusing the caregiver, who can read equivalent values on the patient monitoring system. The display **740**, however, continues to show the plethysmograph **815** (FIG. **8**) and visual pulse indicator **817** (FIG. **8**) waveforms. For one such user setting, the portable alarms remain active.

Another task of the processor **720** includes maintenance of a watchdog function. The watchdog **780** monitors processor status on the watchdog data input **782** and asserts the μP reset output **784** if a fault is detected. This resets the management processor **720**, and the fault is indicated with audible and visual alarms.

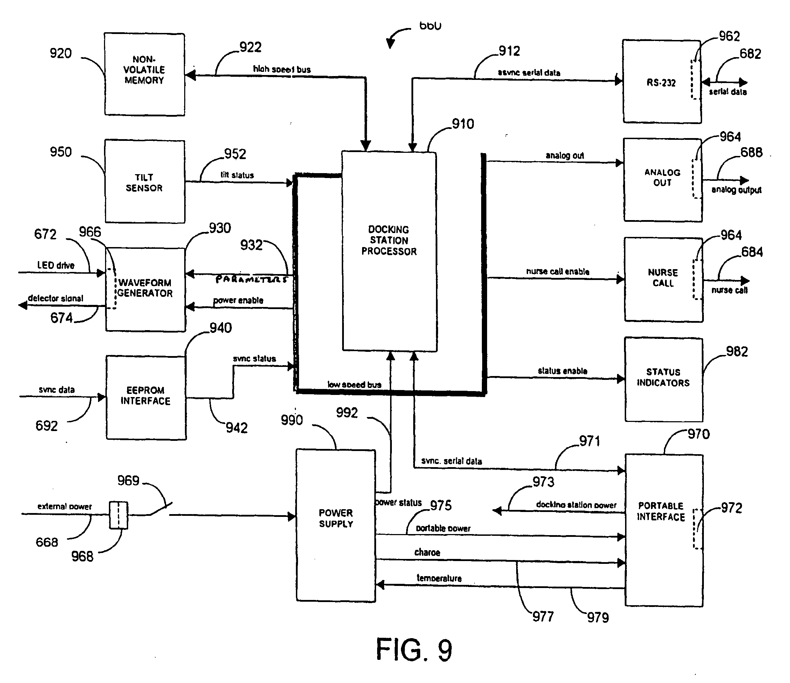

The portable **610** gets its power from batteries in the power supply **730** or from power **766** supplied from the docking station 660 (FIG. **6**) via the docking station interface **760**. A power manager **790** monitors the on/off switch on the keypad **750** and turns-on the portable power accordingly. The power manager **790** turns off the portable on command by the processor **720**. DC/DC converters within the power supply **730** generate the required voltages **738** for operation of the portable **610** and docking station power **732**. The portable batteries can be either alkaline rechargeable batteries or another renewable power source. The batteries of the power supply **730** supply docking station power **732** when the docking station **660** (FIG. **6**) is without external power. A battery charger within the docking station power supply provides charging current **768** to rechargeable batteries within the power supply **730**. The docking station power supply **990** (FIG. **9**) monitors temperature **734** from a thermistor in the rechargeable battery pack, providing an indication of battery charge status.

A non-volatile memory **706** is connected to the management processor **720** via a high-speed bus **722**. In the present embodiment, the memory **706** is an erasable and field re-programmable device used to store boot data, manufacturing serial numbers, diagnostic failure history, adult SpO$_2$ and pulse rate alarm limits, neonate SpO$_2$ and pulse rate alarm limits, SpO$_2$ and pulse rate trend data, and program data. Other types of non-volatile memory are well known. The SpO$_2$ and pulse rate alarm limits, as well as SpO$_2$ related algorithm parameters, may be automatically selected based on the type of sensor **110** (FIG. **2**), adult or neonate, connected to the portable **610**.

The LCD display **740** employs LEDs for a backlight to increase its contrast ratio and viewing distance when in a dark environment. The intensity of the backlight is determined by the power source for the portable **610**. When the portable **610** is powered by either a battery pack within its power supply **730** or a battery pack in the docking station power supply **990** (FIG. **9**), the backlight intensity is at a minimum level. When the portable **610** is powered by external power **668** (FIG. **6**), the backlight is at a higher intensity to increase viewing distance and angle. In one embodiment, button on the portable permits overriding these intensity settings, and provides adjustment of the intensity. The backlight is controlled in two ways. Whenever any key is pressed, the backlight is illuminated for a fixed number of seconds and then turns off, except when the portable is docked and derives power from an external source. In that case, the backlight is normally on unless deactivated with a key on the portable **610**.

FIG. **8A** illustrates the portable user interface **800**, which includes a display **740** and a keypad **750**. In one embodiment, the display **740** is a dot matrix LCD device having **160** pixels by **480** pixels. The display **740** can be shown in portrait mode, illustrated in FIG. **8B**, or in landscape mode, illustrated in FIG. **8C**. A tilt sensor **950** (FIG. **9**) in the docking station **660** (FIG. **6**) or a display mode key on the portable **610** (FIG. **6**) determines portrait or landscape mode. The tilt sensor **950** (FIG. **9**) can be a gravity-activated switch or other device responsive to orientation and can be alternatively located in the portable **610** (FIG. **6**). In a particular embodiment, the tilt sensor **950** (FIG. **9**) is a non-mercury tilt switch, part number CW 1300-1, available from Comus international Nutley, NJ (www.comus-intl.com). The tilt sensor **950** (FIG. **9**) could also be a mercury tilt switch.

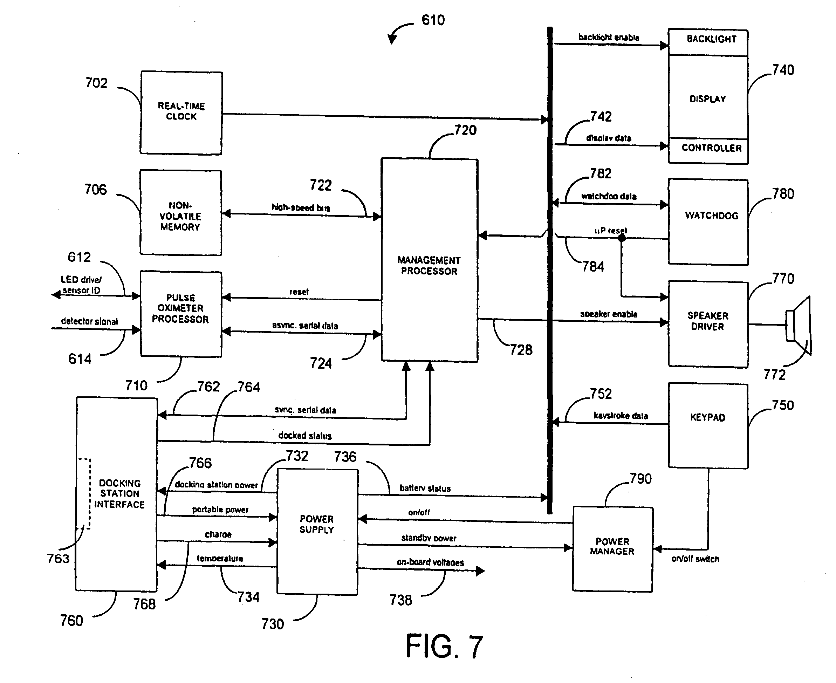

Examples of how the display area can be used to display SpO$_2$ **811**, pulse rate **813**, a plethysmographic waveform **815**, a visual pulse indicator **817** and soft key icons **820** in portrait and landscape mode are shown in FIGS. **8B** and **8C**, respectively. The software program of the management processor **720** (FIG. **7**) can be easily changed to modify the category, layout and size of the display information shown in FIGS. **8B-C**. Other advantageous information for display is SpO$_2$ limits, alarm, alarm disabled, exception messages and battery status.

The keypad **750** includes soft keys **870** and fixed keys **880**. The fixed keys **880** each have a fixed function. The soft keys **870** each have a function that is programmable and indicated by one of the soft key icons **820** located next to the soft keys **870**. That is, a particular one of the soft key icons **820** is in proximity to a particular one of the soft keys **870** and has a text or a shape that suggests the function of that particular one of the soft keys **870**. In one embodiment, the button portion of each key of the keypad **750** is constructed of florescent material so that the keys **870, 880** are readily visible in the dark.

In one embodiment, the keypad **750** has one row of four soft keys **870** and one row of three fixed keys **880**. Other configurations are, of course, available, and specific arrangement is not significant. The functions of the three fixed keys **880** are power, alarm silence and light/contrast. The power function is an on/off toggle button. The alarm silence function and the light/contrast function have dual purposes depending on the duration of the key press. A momentary press of the key corresponding to the alarm silence function will disable the audible alarm for a fixed period of time. To disable the audible alarm indefinitely, the key corresponding to the alarm silence function is held down for a specified length of time. If the key corresponding to the alarm silence function is pressed while the audible alarm has been silenced, the audible alarm is reactivated. If the key corresponding to the light/contrast function is pressed momentarily, it is an onloff toggle button for the backlight. If the key corresponding to the light/contrast function is held down, the display contrast cycles through its possible values.

In this embodiment, the default functions of the four soft keys **870** are pulse beep up volume, pulse beep down volume, menu select, and display mode. These functions are indicated on the display by the up arrow, down arrow, "menu" and curved arrow soft key icons **820**, respectively. The up volume and down volume functions increase or decrease the audible sound or "beep" associated with each detected pulse. The display mode function rotates the display **740** through all four orthogonal orientations, including portrait mode (FIG. **8B**) and landscape mode (FIG. **8C**), with each press of the corresponding key. The menu select function allows the functionality of the soft keys **870** to change from the default functions described above. Examples of additional soft key functions that can be selected using this menu feature are set $SpO_2$ high/low limit, set pulse rate high/low limit, set alarm volume levels, set display to show trend data, print trend data, erase trend data, set averaging time, set sensitivity mode, perform synchronization, perform rechargeable battery maintenance (deep discharge/recharge to remove battery memory), and display product version number.

FIG. **9** provides further details of the docking station **660**, which includes a docking station processor **910**, a non-volatile memory **920,** a waveform generator **930**, a PROM interface **940**, a tilt sensor **950,** a portable interface **970** and associated connector **972**, status indicators **982,** a serial data port **682**, a nurse call output **684**, an analog output **688** and a power supply **990**. In one embodiment, the docking station **660** is intended to be associated with a fixed (non-transportable) host instrument, such as a multiparameter patient monitoring instrument in a hospital emergency room. In a transportable embodiment, the docking station **660** is movable, and includes a battery pack within the power supply **990**.

The docking station processor **910** orchestrates the activity on the docking station **660**. The processor **910** provides the waveform generator **930** with parameters **932** as discussed above for Figures **3** and **4**. The processor **910** also provides asynchronous serial data **912** for communications with external devices and synchronous serial data **971** for communications with the portable **610** (FIG. **6**). In addition, the processor **910** determines system status including sync status **942**, tilt status **952** and power status **992**. The portable management processor **720** (FIG. **7**) performs the watchdog function for the docking station processor **910**. The docking station processor **910** sends watchdog messages to the portable processor **720** (FIG. **7**) as part of the synchronous serial data **972** to ensure the correct operation of the docking station processor **910**.

The docking station processor **910** accesses non-volatile memory **920** over a high-speed bus **922**. The non-volatile memory **920** is re-programmable and contains program data for the processor **910** including instrument communication protocols, synchronization information, a boot image, manufacturing history and diagnostic failure history.

The waveform generator **930** generates a synthesized waveform that a conventional pulse oximeter can process to calculate $SpO_2$ and pulse rate values or exception messages, as described above with respect to FIG. **4**. However, in the present embodiment, as explained above, the waveform generator output does not reflect a physiological waveform. It is merely a waveform constructed to cause the external pulse oximeter to calculate the correct saturation and pulse rate.. In an alternative embodiment, physiological data could be provided to the external pulse oximeter, but the external pulse oximeter would generally not be able to calculate the proper saturation values, and the upgrading feature would be lost. The waveform generator **930** is enabled if an interface cable **690** (FIG. **6**), described below with respect to FIG. **10**, with valid synchronization information is connected. Otherwise, the power to the waveform generator **930** is disabled.

The status indicators **982** are a set of LEDs on the front of the docking station **660** used to indicate various conditions including external power (AC), portable docked, portable battery charging, docking station battery charging and alarm. The serial data port **682** is used to interface with either a computer, a serial port of conventional pulse oximeters or serial printers via a standard RS-232 DB-9 connector **962**. This port **682** can output trend memory, $SpO_2$ and pulse rate and support the system protocols of various manufacturers. The analog output **688** is used to interface with analog input chart recorders via a connector **964** and can output "real-time" or trend $SpO_2$ and pulse rate data. The nurse call output **684** from a connector **964** is activated when alarm limits are exceeded for a predetermined number of consecutive seconds. In another embodiment, data, including alarms, could be routed to any number of communications ports, and even over the Internet, to permit remote use of the upgrading pulse oximeter.

The PROM interface **940** accesses synchronization data **692** from the PROM **1010** (FIG. **10**) in the interface cable **690** (FIGS. **6, 10**) and provides synchronization status **942** to the docking station processor **910**. The portable interface **970** provides the interconnection to the portable **610** (FIG. **6**) through the docking station interface **760** (FIG. **7**).

As shown in FIG. **9**, external power **668** is provided to the docking station **660** through a standard AC connector **968** and on/off switch **969**. When the docking station **660** has external power **668**, the power supply **990** charges the battery in the portable power supply **730** (FIG. **7**) and the battery, if any, in the docking station power supply **990**. When the portable **610** (FIG. **6**) is either removed or turned off, the docking station power **973** is removed and the docking station **660** is turned off, except for the battery charger portion of the power supply **990**. The docking station power **973** and, hence, the docking station **660** turn on whenever a docked portable **610** (FIG. **6**) is turned on. The portable **610** (FIG. **6**) supplies power for an embodiment of the docking station **660** without a battery when external power **668** is removed or fails.

FIG. **10** provides further detail regarding the interface cable **690** used to connect between the docking station **660** (FIG. **6**) and the patient cable **230** (FIG. **2**) of a host instrument **260** (FIG. **2**). The interface cable **690** is configured to interface to a specific host instrument and to appear to the host instrument as a specific sensor. A PROM **1010** built into the interface cable **690** contains information identifying a sensor type, a specific host instrument, and the calibration curve of the specific host instrument. The PROM information can be read by the docking station **660** (FIG. **6**) as synchronization data **692**. Advantageously, the synchronization data **692** allows the docking station **660** (FIG. **6**) to generate a waveform to the host instrument that causes the host instrument to display $SpO_2$ values equivalent to those calculated by the portable **610** (FIG. **6**). The interface cable **690** includes an LED drive path **672**. In the embodiment shown in FIG. **10**, the LED drive path **672** is configured for common anode LEDs and includes IR cathode, red cathode and common anode signals. The interface cable **690** also includes a detector drive path **674**, including detector anode and detector cathode signals.

A menu option on the portable **610** (FIG. **6**) also allows synchronization information to be calculated in the field. With manual synchronization, the docking station **660** (FIG. **6**) generates a waveform to the host instrument **260** (FIG. **2**) and displays an expected $SpO_2$ value. The user enters the $SpO_2$ value displayed on the host instrument using the portable keypad **750** (FIG. **7**). These steps are repeated until a predetermined number of data points are entered and the $SpO_2$ values displayed by the portable and the host instrument are consistent.

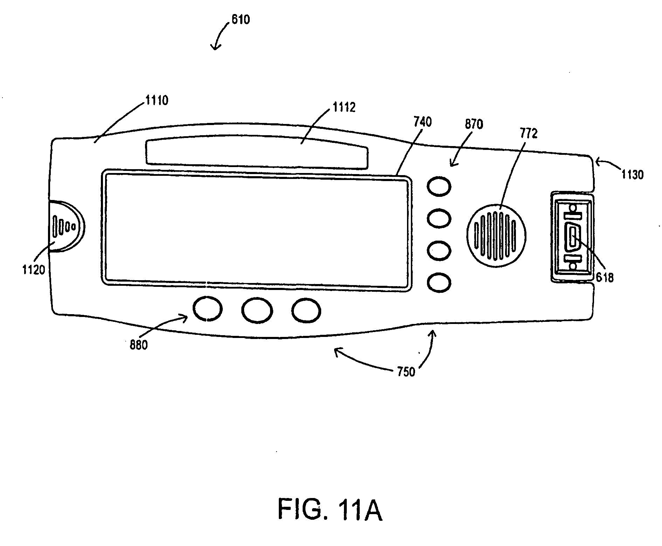

FIG. **11A** depicts an embodiment of the portable **610,** as described above with respect to FIG. **6.**

FIG. **11A** depicts the portable front panel **1110**. The portable **610** has a patient cable connector **618,** as described above with respect to FIG. 6. Advantageously, the connector **618** is rotatably mounted so as to minimize stress on an attached patient cable (not shown). In one embodiment, the connector **618** can freely swivel between a plane parallel to the front panel **1110** and a plane parallel to the side panel **1130**. In another embodiment, the connector **618** can swivel between, and be releasably retained in, three locked positions. A first locked position is as shown, where the connector is in a plane parallel to the front panel **1110.** A second locked position is where the connector **618** is in a plane parallel to the side panel **1130**. The connector **618** also has an intermediate locked position 45° between the first and the second locked positions. The connector **618** is placed in the first locked position for attachment to the docking station **660.**

Shown in FIG. **11A**, the portable front panel **1110** also has a speaker **772**, as described with respect to FIG. **7**. Further, the front panel **1110** has a row of soft keys **870** and fixed keys **880,** as described above with respect to FIG. **8**. In addition, the front panel **1110** has a finger actuated latch **1120** that locks onto a corresponding catch in the docking station **660**, allowing the portable **610** to be releasably retained by the docking station **660**. An OEM label can be affixed to a recessed area **1112** on the front panel **1110.**

The portable back panel has a socket **763**, a pole clamp mating surface, and a battery pack compartment. The socket **763** is configured to mate with a corresponding docking station plug **972.** The socket **763** and plug **972** provide the electrical connection interface between the portable **610** and the docking station **660.** The socket **763** houses multiple spring contacts that compress against plated edge-connector portions of the docking station plug **972**. A conventional pole clamp (not shown) may be removably attached to the mating surface. This conveniently allows the portable **610** to be held to various patient-side or bedside mounts for hands-free pulse oximetry monitoring. The portable power supply **730** (FIG. **7**) is contained within the battery pack compartment. The compartment has a removable cover for protection, insertion and removal of the portable battery pack. Product labels, such as a serial number identifying a particular portable, can be affixed with the back panel indent.

The front side of the docking station **660** has a docking compartment, a pole clamp recess, pivots, a catch, a plug connector **972** and LED status indicators **982**. The docking compartment accepts and retains the portable **610** (FIG. **11A**). When the portable **610** (FIG. **11A**) is docked in the compartment, the pole clamp recess accommodates a pole clamp (not shown) attached to the portable's pole clamp mating surface, assuming the pole clamp is in its closed position. The portable **610** (FIG. **11A**) is retained in the compartment by pivots that fit into corresponding holes in the portable's side face **1130** and a catch that engages the portable's latch **1120** (FIG. **11A**). Thus, the portable **610** (FIG. **11A**) is docked by first attaching it at one end to the pivots, then rotating it about the pivots into the compartment, where it is latched in place on the catch. The portable **610** (FIG. **11A**) is undocked in reverse order, by first pressing the latch **1120** (FIG. **11A**), which releases the portable from the catch, rotating the portable **610** (FIG. **11A**) about the pivots out of the compartment and then removing it from the pivots. As the portable is rotated into the compartment, the docking station plug **972** inserts into the portable socket **763**, providing the electrical interface between the portable **610** and the docking station **660**. The status indicators **982** are as described above with respect to FIG. **9.**

The back side of the docking station **660** has a serial (RS-232 or USB) connector **962**, an analog output and nurse call connector **964**, an upgrade port connector **966**, an AC power plug **968**, an on/off switch **969** and a ground lug. A handle is provided at one end and fan vents are provided at the opposite end. A pair of feet are visible near the back side. A corresponding pair of feet (not visible) are located near the front side. The feet near the front side extend so as to tilt the front side upward, making the display **740** of a docked portable **610** easier to read.

The portable **610** and docking station **660** constitute three distinct pulse oximetry instruments. First, the portable **610** by itself, as depicted in FIG. **11A**, is a handheld pulse oximeter applicable to various patient monitoring tasks requiring battery power or significant mobility, such as ambulance and ER situations. Second, the portable **610** docked in the docking station **660** is a a standalone pulse oximeter applicable to a wide-range of typical patient monitoring situations from hospital room to the operating room. Third, the portable **610** docked and the upgrade port **966** connected with an interface cable to the sensor port of a conventional pulse oximeter module **268** (FIG. **2**) within a multiparameter patient monitoring instrument **260** (FIG. **2**) or other conventional pulse oximeter, is a universal/upgrading pulse oximeter (UPO) instrument **210**, as described herein. Thus, the portable **610** and docking station **660** configuration of the UPO **210** advantageously provides a three-in-one pulse oximetry instrument functionality.

Another embodiment of the docking station **660** incorporates an input port that connects to a blood pressure sensor and an output port that connects to the blood pressure sensor port of a multiparameter patient monitoring system (MPMS). The docking station **660** incorporates a signal processor that computes a blood pressure measurement based upon an input from the blood pressure sensor. The docking station **660** also incorporates a waveform generator connected to the output port that produces a synthesized waveform based upon the computed measurement. The waveform generator output is adjustable so that the blood pressure value displayed on the MPMS is equivalent to the computed blood pressure measurement. Further, when the portable **610** is docked in the docking station **660** and the blood pressure sensor is connected to the input port, the portable displays a blood pressure value according to the computed blood pressure measurement. Thus, in this embodiment, the docking station **660** provides universal/upgrading capability for both blood pressure and $SpO_2$.

Likewise, the docking station **660** can function as an universal/upgrading instrument for other vital sign measurements, such as respiratory rate, EKG or EEG. For this embodiment, the docking station **660** incorporates related sensor connectors and associated sensor signal processors and upgrade connectors to an MPMS or standalone instrument. In this manner, a variety of vital sign measurements can be incorporated into the docking station **660**, either individually or in combination, with or without $SpO_2$ as a measurement parameter, and with or without the portable **610**. In yet another embodiment, the docking station **660** can be configured as a simple $SpO_2$ upgrade box, incorporating a $SpO_2$ processor and patient cable connector for a $SpO_2$ sensor that functions with or without the portable **610**.

Unlike a conventional standalone pulse oximeter, the standalone configuration shown in FIG. **13** has a rotatable display **740** that allows the instrument to be operated in either a vertical or horizontal orientation. A tilt sensor 950 (FIG. 9) indicates when the bottom face is placed along a horizontal surface or is otherwise horizontally-oriented. In this horizontal orientation, the display **740** appears in landscape mode (FIG. **8C**). The tilt sensor **950** (FIG. **9**) also indicates when the side face is placed along a horizontal surface or is otherwise horizontally oriented. In this vertical orientation, the display **740** appears in portrait mode (FIG. **8B**). A soft key **870** on the portable **610** can override the tilt sensor, allowing the display to be presented at any 90° orientation, i.e. portrait, landscape, "upside-down" portrait or "upside-down" landscape orientations. The handheld configuration (FIG. **11A**), can also present the display **740** at any 90° orientation using a soft key **870.** In the particular embodiment described above, however, the portable **610** does not have a tilt sensor and, hence, relies on a soft key **870** to change the orientation of the display when not docked.

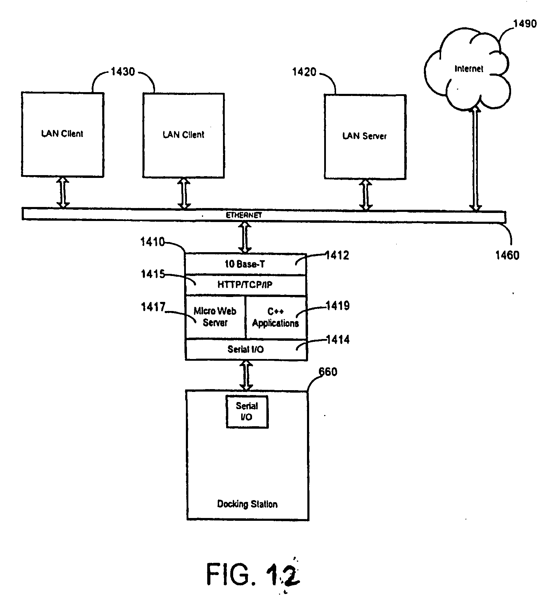

FIG. **12** illustrates the docking station **660** incorporated within a local area network (LAN). The LAN shown is Ethernet-based **1460**, using a central LAN server **1420** to interconnect various LAN clients **1430** and other system resources such as printers and storage (not shown). An Ethernet controller module **1410** is incorporated with the docking station 660. The controller module **1410** can be incorporated within the docking station **660** housing or constructed as an external unit. In this manner, the UPO, according to the present invention, can communicate with other devices on the LAN or over the Internet **1490.**

The Ethernet controller module **1410** can be embedded with web server firmware, such as the Hewlett-Packard (HP) BFOOT-10501. The module **1410** has both a 10 Base-T Ethernet interface for connection to the Ethernet **1460** and a serial interface, such as RS-232 or USB, for connection to the docking station **660.** The module firmware incorporates HTTP and TCP/IP protocols for standard communications over the World Wide Web. The firmware also incorporates a micro web server that allows custom web pages to be served to remote clients over the Internet, for example. Custom C++ programming allows expanded capabilities such as data reduction, event detection and dynamic web page configuration.

As shown in FIG. **12**, there are many applications for the docking station **660** to Ethernet interface.

Multiple UPOs can be connected to a hospital's LAN, and a computer on the LAN could be utilized to upload pulse rate and saturation data from the various UPOs, displaying the results. Thus, this Ethernet interface could be used to implement a central pulse oximetry monitoring station within a hospital. Further, multiple UPOs from anywhere in the world can be monitored from a central location via the Internet. Each UPO is addressable as an individual web site and downloads web pages viewable on a standard browser, the web pages displaying oxygen saturation, pulse rate and related physiological measurements from the UPO. This feature allows a caregiver to monitor a patient regardless of where the patient or caregiver is located. For example a caregiver located at home in one city or at a particular hospital could download measurements from a patient located at home in a different city or at the same or a different hospital. Other applications include troubleshooting newly installed UPOs or uploading software patches or upgrades to UPOs via the Internet. In addition alarms could be forwarded to the URL of the clinician monitoring the patient.

The UPO may have other configurations besides the handheld unit described in connection with FIG. **5** or the portable **610** and docking station **660** combination described in connection with FIG. **11**. The UPO may be a module, with or without a display, that can be removably fastened to a patient via an arm strap, necklace or similar means. In a smaller embodiment, this UPO module may be integrated into a cable or connector used for attaching a sensor to a pulse oximeter. The UPO may also be a circuit card or module that can externally or internally plug into or mate with a standalone pulse oximeter or multiparameter patient monitoring system. Alternatively, the UPO may be configured as a simple standalone upgrade instrument.

Further, although a universal/upgrading apparatus and method have been mainly described in terms of a pulse oximetry measurement embodiment, the present invention is equally applicable to other physiological measurement parameters such as blood pressure, respiration rate, EEG and ECG, to name a few. In addition, a universal/upgrading instrument having a single physiological measurement parameter or a multiple measurement parameter capability and configured as a handheld, standalone, portable, docking station, module, plug-in, circuit card, to name a few, is also within the scope of the present invention.

The UPO has been disclosed in detail in connection with various embodiments of the present invention. These embodiments are disclosed by way of examples only and are not to limit the scope of the present invention, which is defined by the claims that follow. One of ordinary skill in the art will appreciate many variations and modifications within the scope of this invention.

**Claims**

1. A physiological- measurement system capable of measuring one or more physiological parameters, the system comprising:

   to first pulse oximeter (310) configured to determine oxygen saturation measurements and pulse rate measurements based upon an intensity signal responsive to attenuation of light at a tissue site; and
   waveform generation means (320) in communication with the first pulse oximeter (310), the waveform generation means (320) configured to provide a synthesized waveform to a sensor port (262) of a second pulse oximeter (260) based upon said oxygen saturation measurement and said pulse rate measurement, wherein the synthesized waveform is configured to cause the second pulse oximeter (260) to display an oxygen saturation and pulse rate generally equivalent to that measured by the first pulse oximeter (310).

2. The measurement system of Claim 1 further comprising means (740) for displaying said oxygen saturation measurement and said pulse rate measurement.

3. The measurement system of Claim 2 further comprising means (950) for sensing gravity and for altering an orientation mode of said means for displaying as a function of the orientation of said first pulse oximeter.

4. The measurement system of Claim 2 further comprising means(972) for manually altering an orientation mode of said display means.

**Patentansprüche**

1. Physiologisches Messsystem, das einen oder mehrere physiologische Parameter messen kann, wobei das System aufweist:

   ein erstes Pulsoximeter (310), das so konfiguriert ist, dass es Sauerstoffsättigungsmessungen und Pulsfrequenzmessungen auf der Grundlage eines Intensitätssignals als Reaktion auf Lichtdämpfung an einer Gewebestelle bestimmt; und
   ein Wellenformerzeugungsmittel (320) in Kommunikation mit dem ersten Pulsoximeter (310), wobei das Wellenformerzeugungsmittel (320) so konfiguriert ist, dass es eine synthetische Wellenform auf der Grundlage der Sauerstoffsättigungsmessung und der Pulsfrequenzmessung einem Sensorport (262) eines zweiten Pulsoximeters (260) zuführt, wobei die synthetische Wellenform so konfiguriert ist, dass sie das zweite Pulsoximeter (260) veranlasst, eine Sauerstoffsättigung und Pulsfrequenz anzuzeigen, die zu der durch das erste Pulsoximeter (310) gemessenen allgemein äquivalent ist.

2. Messsystem nach Anspruch 1, ferner mit einem Mittel (740) zum Anzeigen der Sauerstoffsättigungsmessung und der Pulsfrequenzmessung.

3. Messsystem nach Anspruch 2, ferner mit einem Mittel (950) zum Erfassen der Schwerkraft und zum Ändern eines Orientierungsmodus des Mittels zum Anzeigen als Funktion der Orientierung des ersten Pulsoximeters.

4. Messsystem nach Anspruch 2, ferner mit einem Mittel (972) zum manuellen Ändern eines Orientierungsmodus des Anzeigemittels.


**Revendications**

1. Système de mesure physiologique apte à mesurer un ou plusieurs paramètres physiologiques, ledit système comprenant :

   un premier oxymètre de pouls (310) prévu pour déterminer des mesures de saturation en oxygène et des mesures de pouls sur la base d'un signal d'intensité en réaction à une atténuation de lumière sur un site tissulaire ; et
   un moyen générateur de forme d'onde (320) relié au premier oxymètre de pouls (310), ledit moyen générateur de forme d'onde (320) étant prévu pour émettre une forme d'onde synthétisée vers un port de capteur (262) d'un deuxième oxymètre de pouls (260) sur la base de la mesure de saturation en oxygène et de la mesure de pouls, la forme d'onde synthétisée étant prévue pour provoquer l'indication par le deuxième oxymètre de pouls (260) d'un taux de saturation en oxygène et d'un pouls généralement équivalents à ceux mesurés par le premier oxymètre de pouls (310).

2. Système de mesure selon la revendication 1, comprenant en outre un moyen (740) d'affichage de la mesure de saturation en oxygène et de la mesure de pouls.

3. Système de mesure selon la revendication 2, comprenant en outre un moyen (950) de détection de gravité et de commutation d'un mode d'orientation du moyen d'affichage en tant que fonction de l'orientation du premier oxymètre de pouls.

4. Système de mesure selon la revendication 2, comprenant en outre un moyen (972) de commutation manuelle d'un mode d'orientation du moyen d'affichage.

PULSE OXIMETER

SENSOR

FIG. 1 (prior art)

**FIG. 2**

EP 1 889 569 B1

FIG. 3

16

FIG. 4

EP 1 889 569 B1

FIG. 5

FIG. 6

FIG. 7

FIG. 8C

FIG. 8B

FIG. 8A

FIG. 9

EP 1 889 569 B1

690

674

detector anode

detector cathode

inner shield

672

IR cathode

red cathode

common anode

Vcc

1010

sync data

PROM

692

outer shield

EP 1 889 569 B1

FIG. 10

FIG. 11A

FIG. 12

EP 1 889 569 B1

**EP 1 889 569 B1**

## REFERENCES CITED IN THE DESCRIPTION

**Patent documents cited in the description**

- US 5687717 A
- US 5632272 A
- US 5638818 A
- US 5758644 A